# DEMANDE DE BREVET EUROPEEN

(11) **EP 1 566 164 A1**
(43) Date de publication de la demande: **24.08.2005**
(21) Numéro de dépôt: 05290178.2
(22) Date de dépôt: 27.01.2005
(51) Int. Cl.: A61K 7/06

(54) **Procédé de préparation d'une composition pour le traitement cosmétique des matières kératiniques à partir de fluide sous pression et de polymères anioniques et/ou non ioniques**

(30) Priorité: 29.01.2004 FR 0400853
(71) Demandeur: L'OREAL, 75008 Paris (FR)
(72) Inventeur: De la Mettrie, Roland, 78110 Le Vésinet (FR)
(74) Mandataire: Casalonga, Axel

(57) **Abrégé**

L'invention concerne un procédé de préparation d'une composition cosmétique pour le traitement cosmétique des matières kératiniques, caractérisé en ce qu'il comprend une étape de percolation de vapeur d'eau sous une pression d'au moins 3 bars au travers d'au moins un polymère anionique et/ou un polymère non ionique, sous forme solide.

## Description

La présente invention a pour objet un procédé de préparation d'une composition destinée au traitement cosmétique des matières kératiniques, en particulier de la peau et des fibres kératiniques humaines telles que les cheveux, ainsi qu'un procédé de traitement cosmétique des matières kératiniques à partir de cette composition.

En cosmétique, on cherche toujours à améliorer les propriétés cosmétiques des matières kératiniques, et notamment les propriétés coiffantes des cheveux. On peut alors leur appliquer des compositions de traitement cosmétique comprenant des agents coiffants tels que des polymères anioniques ou non ioniques, destinés à conférer de la brillance, de la légèreté, du volume et du maintien aux cheveux et à faciliter leur démêlage.

Cependant, les compositions de traitement cosmétique contenant de tels agents coiffants sont généralement des compositions aqueuses dans lesquelles lesdits agents doivent être solubilisés. Le manque de solubilité de ces agents coiffant diminue le pouvoir coiffant de ces compositions. En outre, ce critère de solubilité réduit le nombre d'agents coiffants que l'on peut utiliser pour le traitement cosmétique des matières kératiniques. Par ailleurs, certains polymères fixants contiennent des monomères à motifs esters ou sont des polyesters issus de la polycondensation de diols et de diacides. Ce sont alors des composés sensibles à l'hydrolyse en particulier en milieux acides ou alcalins, ce qui limite leurs possibilités de formulation.

La Demanderesse a découvert de manière surprenante qu'en utilisant un nouveau procédé de préparation d'une composition de traitement cosmétique des matières kératiniques, on pouvait obtenir en un temps très court, inférieur à 2 minutes, des compositions plus ou moins concentrées en agent(s) coiffant(s) selon le besoin, notamment sans conservateur, permettant de surmonter les problèmes de solubilité et de stabilité exposés ci-dessus.

Ce procédé est mis en oeuvre simplement. On fait passer un fluide sous pression, dont la température est de préférence supérieure ou égale à 30°C, et encore plus préférentiellement allant de 30°C à 150°C, mieux encore de 40°C à 120°C, pendant un laps de temps très court, inférieur à 1 minute, à travers au moins un polymère anionique et/ou un polymère non ionique, sous forme solide ou pâteuse, de préférence sous forme solide, et encore de préférence sous forme pulvérulente.

Il permet d'utiliser sous forme anhydre des polymères anioniques et/ou non ioniques instables dans des compositions aqueuses, soit parce qu'ils réagissent avec l'eau, soit parce qu'ils réagissent en solution aqueuse avec des composés qui ne réagissent pas avec eux dans une composition anhydre.

Les compositions préparées selon ce procédé peuvent présenter une stabilité au stockage limitée, ce qui n'est pas ici un inconvénient puisque le procédé conduit à une composition prête à l'emploi, destinée à être utilisée rapidement après sa préparation, par exemple dans les 5 minutes suivant sa préparation, notamment après refroidissement jusqu'à une température cosmétiquement acceptable, de préférence inférieure à 60°C. La composition peut être utilisée jusqu'à une semaine après sa préparation, selon la vitesse de dégradation du polymère anionique et/ou non ionique.

Etant donné le temps de préparation très court, les compositions de traitement cosmétique peuvent être préparées "à la demande" en mélangeant différents composés actifs en cosmétique selon les propriétés cosmétiques recherchées.

Selon un autre mode de réalisation, les polymères anioniques et/ou non ioniques peuvent être conditionnés dans un dispositif prêt-à-l'emploi, et il n'est pas nécessaire de déterminer au préalable les concentrations des composés actifs en solution, ce qui limite les erreurs de mesure de l'utilisateur.

En outre, le procédé selon l'invention permet d'éviter l'utilisation de flacons multi-compartiments, ce qui rend le procédé particulièrement économique et plus sûr pour l'utilisateur.

La composition ainsi obtenue peut être utilisée seule ou en mélange avec une autre composition.

Un avantage important de ce procédé de préparation est l'obtention de compositions conférant de bonnes propriétés cosmétiques. En particulier, les fibres kératiniques traitées avec une composition obtenue par le procédé selon l'invention présentent des propriétés coiffantes améliorées, et notamment de démêlage, de brillance, de légèreté, d'aspect visuel, de volume, et de maintien.

L'invention a donc pour objet un procédé de préparation d'une composition de traitement cosmétique des matières kératiniques caractérisé en ce qu'il comprend une étape de percolation d'un fluide sous une pression d'au moins 3 bars au travers d'au moins un polymère anionique et/ou un polymère non ionique, sous forme solide ou pâteuse.

Un autre objet de l'invention est une composition susceptible d'être obtenue par le procédé selon l'invention.

L'invention a également pour objet l'utilisation de la composition obtenue selon le procédé de l'invention pour le traitement cosmétique des matières kératiniques, et notamment le coiffage des cheveux.

L'invention a enfin pour objet un dispositif de conditionnement permettant de mettre en oeuvre le procédé de préparation de la présente invention.

D'autres objets, caractéristiques, aspects et avantages de l'invention apparaîtront encore plus clairement à la lecture de la description et des exemples qui suivent.

Selon l'invention, le procédé de préparation d'une composition de traitement cosmétique des matières kératiniques comprend une étape de percolation d'un fluide de préférence à une température supérieure ou égale à 30°C, mieux encore allant de 30°C à 150°C, mieux encore de 40°C à 120°C, sous une pression d'au moins 3 bars (3.10⁵ Pa), au travers d'au moins un polymère anionique et/ou un polymère non ionique, sous forme solide ou pâteuse.

La percolation est un mouvement de fluide à travers un milieu poreux saturé permettant le passage du fluide, sous l'action ou l'effet de la pression.

Le fluide peut être constitué par de la vapeur d'eau éventuellement accompagnée d'eau liquide, ou par un ou plusieurs solvants liquides et/ou gazeux cosmétiquement acceptables, notamment organiques, ou encore par un mélange de vapeur d'eau éventuellement accompagnée d'eau liquide, et d'un ou plusieurs solvants liquides et/ou gazeux cosmétiquement acceptables. De préférence, le fluide comprend au moins de la vapeur d'eau pouvant être accompagnée d'eau liquide, et encore plus préférentiellement il est de la vapeur d'eau pouvant être accompagnée d'eau liquide.

A titre de solvant organique, on peut par exemple citer les alcools inférieurs en C1-C4, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Par « composé insoluble dans l'eau », on entend tout composé qui, à une concentration supérieure ou égale à 0,1% en poids dans l'eau à 25°C, ne forme pas à l'oeil nu une solution isotrope transparente.

Le polymère anionique et/ou le polymère non ionique est sous forme solide ou sous forme pâteuse, de préférence sous forme solide, et encore plus préférentiellement sous forme pulvérulente.

Par « forme pâteuse » au sens de la présente invention, on entend une consistance intermédiaire entre une phase solide et une phase liquide. La viscosité de cette phase pâteuse est de préférence supérieure à 0,1 Pa.s, et encore plus préférentiellement supérieure à 1 Pa.s, ceci à 25°C avec un taux de cisaillement de 10 s⁻¹.

Par "matières kératiniques", on entend la peau, les muqueuses telles que les lèvres, et/ou les phanères tels que les ongles et les fibres kératiniques, par exemple les cils, les sourcils et les cheveux.

Le procédé de la présente invention peut être mis en oeuvre à partir d'un dispositif classique permettant de générer un fluide sous pression, à une température de préférence supérieure ou égale à 30°C, et encore plus préférentiellement allant de 30°C à 150°C. Un tel dispositif comprend une chambre résistant à la pression, équipée d'un bloc thermique, ainsi qu'un circuit d'acheminement du fluide produit vers le polymère anionique et/ou non ionique.

Selon un autre mode de réalisation, le dispositif comprend de plus un réservoir de liquide(s) ainsi qu'une pompe permettant l'acheminement du ou des liquides vers la chambre.

Le liquide contenu dans le réservoir est soit de l'eau, soit un solvant cosmétiquement acceptable ou un mélange de plusieurs solvants cosmétiquement acceptables, ou encore un mélange d'eau et d'un ou plusieurs solvants cosmétiquement acceptables. De préférence, le liquide comprend au moins de l'eau, et encore plus préférentiellement il est de l'eau.

Un dispositif particulièrement utile pour la mise en oeuvre du procédé de la présente invention est une machine à café du type « expresso ». De telles machines sont bien connues de la technique. Par exemple, ces machines sont décrites dans les brevets AT 168405, US 2688911, DE 32433870 et IT 1265636.

Selon un mode de réalisation particulier de l'invention, l'étape de percolation est mise en oeuvre avec un fluide à une température supérieure ou égale à 30°C, de préférence allant de 30°C à 150°C, sous une pression comprise entre 3 et 30 bars, ou d'au moins 4 bars, de préférence supérieure à 10 bars et tout particulièrement comprise entre 10 et 30 bars.

Selon un mode de réalisation particulier de l'invention, l'étape de percolation est mise en oeuvre avec de la vapeur d'eau sous une pression d'au moins 4 bars, de préférence supérieure à 10 bars et tout particulièrement comprise entre 10 et 30 bars.

Le ou les polymères anionique et/ou non ioniques, sous forme solide ou pâteuse, peuvent être utilisés directement dans le dispositif générant le fluide sous pression dans un récipient destiné à cet usage. Ils peuvent aussi être conditionnés dans un dispositif de conditionnement d'une composition cosmétique particulier comprenant un logement fermé délimité par au moins une paroi au moins en partie perméable au fluide sous une pression d'au moins 3 bars, la composition contenant au moins un polymère anionique et/ou un polymère non ionique. De tels dispositifs sont, par exemple, décrits dans les demandes de brevets WO 00/56629, EP512470 ou WO 99/03753.

Selon un mode de réalisation particulier, le logement est délimité par deux feuilles scellées. Selon un autre mode de réalisation, le logement est délimité par une barquette fermée par un couvercle.

Ces dispositifs peuvent être fabriqués à partir de matériaux tissés ou non tissés, en matière plastique ou végétale, par exemple en cellulose, en métal tel que l'aluminium ou en composite. De tels dispositifs sont par exemple décrits dans les demandes de brevets WO 00/56629, EP512470 ou WO 99/03753.

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono- ou diacides carboxyliques insaturés tels que ceux répondant à la formule : 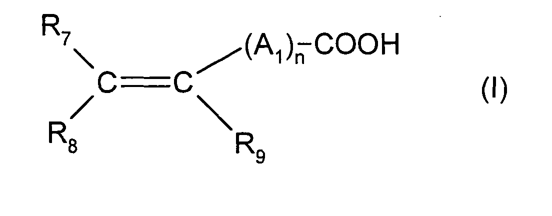 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels, les copolymères d'acide acrylique et d'acrylamide, les sels de sodium des acides polyhydroxycarboxyliques.
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois n^{os} 75370 et 75371. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, et les terpolymères acide méthacrylique/acrylate d'éthyle/acrylate de tertiobutyle.
   On peut aussi citer les copolymères acide méthacrylique/acide acrylique/acrylate d'éthyle/méthacrylate de méthyle en dispersion aqueuse.
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch.
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805.
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.

   Ces polymères sont par exemple décrits dans les brevets français n^{os} 2 350 384 et 2 357 241 de la demanderesse.
E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone.
- les sels de l'acide polystyrène-sulfonique. Ces composés sont décrits dans le brevet FR 2 198 719.
- les sels d'acides polyacrylamide-sulfoniques tels que ceux mentionnés dans le brevet US 4 128 631, et plus particulièrement l'acide polyacrylamidoéthylpropane-sulfonique.

On peut également citer les polyuréthanes non ioniques.

Selon l'invention, les polymères fixants anioniques sont de préférence choisis parmi les copolymères d'acide acrylique tels que les terpolymères acide acrylique/acrylate d'éthyle/N-tertiobutylacrylamide, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié, les copolymères d'acide méthacrylique et de méthacrylate de méthyle, les copolymères d'acide méthacrylique et d'acrylate d'éthyle et les copolymères acétate de vinyle/acide crotonique et les copolymères acétate de vinyle/acide crotonique greffés par du polyéthylèneglycol.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle;
- les copolymères d'acrylonitrile et d'un monomère non ionique choisis, par exemple, parmi le butadiène et les (méth)acrylates d'alkyle ;
- les homopolymères de styrène ;
- les copolymères de styrène comme, par exemple, les copolymères de styrène et de (méth)acrylate d'alkyle ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, et
- les copolymères de vinyllactame, les copolymères poly(vinylpyrrolidone/acétate de vinyle), et les terpolymères poly(vinylpyrrolidone/acétate de vinyle/propionate de vinyle)
- les polyuréthanes non ioniques.

Les groupes alkyle des polymères non ioniques mentionnés ci-dessus ont, de préférence, de 1 à 6 atomes de carbone.

Le ou les polymères anionique et/ou non ioniques selon l'invention peuvent être mis en oeuvre en mélange avec un ou plusieurs adjuvants solides ou pâteux, et de préférence pulvérulents. Les adjuvants peuvent être choisis parmi les argiles, les sels, les agents tensioactifs anioniques, cationiques, non-ioniques, amphotères, zwittérioniques, les épaississants naturels ou de synthèse, les billes de verre, la silice, le nylon, l'alumine, le dioxyde de titane, les zéolithes, le chitosane, la maltodextrine, la cyclodextrine, les mono ou disaccharides comme le glucose, le saccharose, le sorbitol, le fructose, l'oxyde de zinc, de zirconium, les particules de résine comme la silicone ou les silicabades, le talc, les borosilicates notamment de calcium, le polyéthylène, le coton, le polytétrafluoroéthylène (PTFE), la cellulose et ses dérivés, les composés superabsorbants, les carbonates de magnésium ou de calcium, l'amidon éventuellement modifié, les grains de maïs, les gommes de polydiméthylsiloxane, l'hydroxyapatite poreux, la soie, le collagène, la sciure de bois, la poudre de fucus, les farines ou extraits de blé, de riz, de pois, de lupin, de soja, d'orge, l'alginate de calcium, le charbon actif, les particules de poly(chlorure de vinylidène/acrylonitrile), notamment celles commercialisées sous la dénomination générale d' « Expancel® » par la société AKZO NOBEL sous les références particulières « Expancel® WE » ou « DE » Expancels, et leurs mélanges.

Lorsqu'un ou plusieurs adjuvants sont présents, le ou les polymères anioniques et/ou non ioniques de l'invention sont présents de préférence en une quantité allant de 0,5 à 99% en poids, mieux encore de 1 à 80 % en poids, et encore plus préférentiellement de 2 à 60 % en poids par rapport au poids total polymères anioniques et/ou non ioniques et adjuvants.

Les plantes ou extraits de plantes utilisés et au travers desquels passe le fluide sous pression peuvent être soumis avant la percolation à un traitement tel qu'une torréfaction, un cryobroyage, ou une lyophilisation.

La composition de traitement cosmétique des matières kératiniques obtenue selon le procédé de l'invention contient outre le ou les polymères anioniques et/ou non ioniques et le(s) composant(s) du fluide, à savoir l'eau et/ou le(s) solvant(s) cosmétiquement acceptable(s), éventuellement tout ou partie du ou des adjuvants présents dans le mélange solide ou pâteux.

L'invention concerne également une composition susceptible d'être obtenue par le procédé selon l'invention, la composition étant de préférence exempte d'agents conservateurs.

A partir du procédé de préparation de l'invention, on obtient une composition de traitement cosmétique des matières kératiniques qui peut être appliquée directement sur les matières kératiniques, ou qui peut être mélangée à un milieu cosmétiquement acceptable, ou encore au moins un additif classiquement utilisé en cosmétique pourra y être ajouté par un opérateur. On peut également mélanger au moins deux compositions obtenues par le procédé de l'invention. La composition de traitement cosmétique des matières kératiniques résultant éventuellement de mélange(s) et/ou addition(s) indiqués ci-dessus, sera dénommée ci-après composition finale de traitement cosmétique ou composition finale.

Un mode de réalisation particulier de l'invention consiste à appliquer la composition obtenue par l'intermédiaire d'un dispositif ne nécessitant pas d'intervention humaine et équipé éventuellement d'un moyen de refroidissement.

La quantité du ou des polymères anioniques et/ou non ioniques présents dans la composition finale de traitement cosmétique obtenue par le procédé de la présente invention est en général comprise entre 0,001 et 50 % en poids environ du poids total de la composition finale de traitement cosmétique, de préférence entre 0,005 et 30 %, et encore plus préférentiellement entre 0,01 et 20%.

Lorsque la composition de traitement cosmétique obtenue par le procédé de la présente invention est mélangée à un milieu cosmétiquement acceptable, un tel milieu est généralement constitué par de l'eau ou par un mélange d'eau et d'au moins un solvant organique pour solubiliser les composés qui ne seraient pas suffisamment solubles dans l'eau.

A titre de solvant organique, on peut citer, par exemple, les alcools inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40% en poids par rapport au poids total de la composition finale, et encore plus préférentiellement entre 5 et 30% en poids.

Au moins un additif classiquement utilisé en cosmétique peut être également ajouté dans les compositions de traitement cosmétique obtenues selon le procédé de la présente invention. A titre d'exemples de tels additifs, on peut citer des agents tensioactifs anioniques, cationiques, non-ioniques, amphotères, zwittérioniques ou leurs mélanges, des polymères cationiques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que, par exemple, des huiles de silicone, des agents filmogènes, des céramides, des agents conservateurs, des agents opacifiants, mais aussi des huiles, des cires, des gommes, des pigments colorés ou nacrés.

Les additifs ci-dessus sont en général présents en quantité comprise pour chacun d'eux entre 0,01 et 20% en poids par rapport au poids de la composition finale.

Bien entendu, l'homme de l'art veillera à choisir ce ou ces éventuels composés complémentaires de manière telle que les propriétés avantageuses attachées intrinsèquement à la composition de traitement cosmétique conforme à l'invention ne soient pas, ou substantiellement pas, altérées par la ou les adjonctions envisagées.

Le pH de la composition finale est généralement compris entre 3 et 12, et de préférence entre 5 et 11. Il peut être ajusté à la valeur désirée au moyen d'agents acidifiants ou alcalinisants habituellement utilisés en cosmétique ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 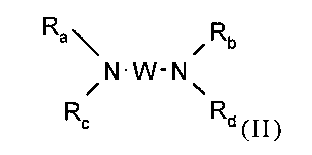 dans laquelle W est un reste propylène éventuellement substitué par un groupement hydroxyle ou un radical alkyle en C1-C4 ; Ra, Rb, Rc et Rd, identiques ou différents, représentent un atome d'hydrogène, un radical alkyle en C1-C4 ou hydroxyalkyle en C1-C4.

La composition finale de traitement cosmétique peut se présenter sous des formes diverses, telles que sous forme de liquides, de crèmes, de mascara, de gels ou sous toute autre forme appropriée pour réaliser un traitement des matières kératiniques, et notamment des fibres kératiniques, et de la peau.

La composition finale de traitement cosmétique peut être utilisée, par exemple, comme shampoing, après-shampoing, soin rincé ou non rincé, masque de soin profond, gel douche, lotion ou crème de traitement des matières kératiniques.

La présente invention concerne aussi un procédé de traitement cosmétique des matières kératiniques, comprenant la préparation d'une composition de traitement cosmétique selon le procédé tel que défini ci-dessus, et son application sur les matières kératiniques, par exemple par l'intermédiaire d'un opérateur ou par l'intermédiaire d'un dispositif ne nécessitant pas d'intervention humaine. La durée d'application peut varier entre 15 secondes et 1 heure.

Avant application, la composition de traitement cosmétique obtenue selon le procédé de l'invention, peut être mélangée à un milieu cosmétiquement acceptable et/ou à un ou plusieurs additifs classiquement utilisés en cosmétique tels que décrits ci-dessus.

Un autre mode de réalisation consiste à préparer au moins deux compositions de traitement cosmétique selon le procédé de l'invention, à les mélanger, et à ajouter éventuellement un milieu cosmétiquement acceptable et/ou un ou plusieurs additifs classiquement utilisés en cosmétique tels que décrits ci-dessus, puis à appliquer la composition finale obtenue sur les matières kératiniques.

Les exemples ci-après sont destinés à illustrer la présente invention.

### Exemple 1

On mélange les ingrédients suivants dans les proportions indiquées en % en poids par rapport au poids total de mélange solide :
- copolymère vinylpyrrolidone / acétate de vinyle (65/65) vendu sous la dénomination commerciale PVP/VA S 630 L par la société ISP 70 %
- cetyl hydroxyéthylcellulose vendu sous la dénomination commerciale NATROSL PLUS, GRADE 330 par la société AQUALON 30 %

On place 5 g de ce mélange dans une machine expresso du commerce. On fait ensuite passer de la vapeur d'eau jusqu'à l'obtention d'une composition (A) présentant un volume final de 50 ml.

On peut ensuite ajouter à deux parties en poids de composition (A), une partie en poids d'une composition aqueuse (B) contenant 1 % en poids d'hydroxyéthylcellulose, pour faciliter l'application.

On obtient ainsi une composition de traitement cosmétique prête à être appliquée sur les cheveux.

On obtient des cheveux légers, brillants, d'aspect agréable et facilement démêlables.

### Exemple 2

On mélange les ingrédients suivants dans les proportions indiquées en % en poids par rapport au poids total de mélange solide :
- poly diméthyl / méthyl siloxane à groupements propyl thio-3 acrylate de méthyle / méthacrylate de méthyle / acide méthacrylique vendu sous la dénomination commerciale VS 80 DRY par la société 3M 40 %
- carboxyméthyl cellulose de sodium vendu sous la dénomination commerciale BLANOSE 7M8SF par la société AQUALON 45%
- D-xylose 10%
- cetyl hydroxyéthylcellulose vendu sous la dénomination commerciale NATROSL PLUS, GRADE 330 par la société AQUALON 5 %
   On place 5 g de ce mélange dans une machine expresso du commerce. On fait ensuite passer de la vapeur d'eau jusqu'à l'obtention d'une composition (A) présentant un volume final de 50 ml.
   On peut ensuite ajouter à deux parties en poids de composition (A), une partie en poids d'une composition aqueuse (B) contenant 1 % en poids d'hydroxyéthylcellulose, pour faciliter l'application.
   On obtient ainsi une composition de traitement cosmétique prête à être appliquée sur les cheveux.
   On obtient des cheveux légers, brillants, d'aspect agréable et facilement démêlables.

## Revendications

1. Procédé de préparation d'une composition pour le traitement cosmétique des matières kératiniques, **caractérisé en ce qu'**il comprend une étape de percolation d'un fluide sous une pression d'au moins 3 bars au travers d'au moins un polymère anionique et/ou un polymère non ionique, sous forme solide ou pâteuse.

2. Procédé selon la revendication 1, **caractérisé en ce que** le polymère anionique est choisi parmi les polymères comportant des groupes dérivés d'acides carboxylique, sulfonique ou phosphorique, et présentant une masse moléculaire en poids compris entre 500 et 5000000, et leurs mélanges.

3. Procédé selon la revendication 1, **caractérisé en ce que** le polymère non ionique est choisi parmi les homopolymères de vinylpyrrolidone, les copolymères de vinylpyrrolidone et d'acétate de vinyle, les polyalkyloxazolines, les homopolymères d'acétate de vinyle, les copolymères d'acétate de vinyle et d'ester acrylique, les copolymères d'acétate de vinyle et d'éthylène, les copolymères d'acétate de vinyle et d'ester maléique, les copolymères de polyéthylène et d'anhydride maléique, les homopolymères d'acrylates d'alkyle et les homopolymères de méthacrylates d'alkyle, les copolymères d'esters acryliques, les copolymères d'acrylonitrile et de butadiène ou de (méth)acrylate d'alkyle, les polyuréthanes, les copolymères d'acétate d'alkyle et d'uréthane, les polyamides, et leurs mélanges.

4. Procédé selon l'une quelconque des revendications précédentes, **caractérisé en ce que** le ou les polymères anionique et/ou non ioniques sous forme solide ou pâteuse sont mis en oeuvre en mélange avec au moins un adjuvant.

5. Procédé selon la revendication 4, **caractérisé en ce que** l'adjuvant est choisi parmi les argiles, les sels, les tensioactifs anioniques, non ioniques, cationiques ou zwittérioniques, les épaississants naturels ou de synthèse, l'amidon, éventuellement modifié, les billes de verre, la silice, le nylon, l'alumine, le dioxyde de titane, les zéolithes, le chitosane, la maltodextrine, la cyclodextrine, les mono ou disaccharides, l'oxyde de zinc, de zirconium, les particules de résine comme la silicone ou les silicabades, le talc, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, les gommes de polydiméthylsiloxane, l'hydroxyapatite poreux, la soie, le collagène, la sciure de bois, la poudre de fucus, les farines ou extraits de blé, de riz, de pois, de lupin, de soja, d'orge, l'alginate de calcium, le charbon actif, et les particules de poly(chlorure de vinylidène/acrylonitrile).

6. Procédé selon la revendication 4 ou 5, **caractérisé en ce que** le ou les polymères anioniques et/ou non ioniques sont présents en une quantité allant de 0,5 à 99% en poids, de préférence de 1 à 80 % et encore plus préférentiellement de 2 à 60% en poids par rapport au poids total polymère(s) anionique(s) et/ou non ionique(s) et adjuvant.

7. Procédé selon l'une quelconque des revendications précédentes, **caractérisé en ce que** l'étape de percolation est mise en oeuvre avec un fluide sous une pression comprise entre 3 et 30 bars, de préférence comprise entre 10 et 30 bars.

8. Procédé selon l'une quelconque des revendications précédentes, **caractérisé en ce que** l'étape de percolation est mise en oeuvre avec un fluide sous pression d'au moins 10 bars.

9. Procédé selon l'une quelconque des revendications précédentes, **caractérisé en ce que** le fluide est de la vapeur d'eau, éventuellement accompagnée d'eau liquide.

10. Procédé selon l'une quelconque des revendications 1 à 8, **caractérisé en ce que** le fluide est constitué par un ou plusieurs solvants organiques liquides et/ou gazeux cosmétiquement acceptables.

11. Composition de traitement cosmétique des matières kératiniques susceptible d'être obtenue par la procédé selon l'une quelconque des revendications précédentes.

12. Composition selon la revendication 11, **caractérisée en ce qu'**elle est exempte d'agent conservateur.

13. Procédé de traitement cosmétique des matières kératiniques, **caractérisé en ce qu'**on prépare une composition cosmétique selon le procédé défini selon l'une quelconque des revendications 1 à 10 et qu'on applique cette composition sur la matière kératinique.

14. Procédé de traitement cosmétique des matières kératiniques selon la revendication 13, **caractérisé en ce qu'**on applique la composition sur la matière kératinique par l'intermédiaire d'un dispositif ne nécessitant pas d'intervention humaine.

15. Procédé de traitement cosmétique des matières kératiniques selon la revendication 13, **caractérisé en ce que**, avant l'application, la composition de traitement cosmétique préparée selon le procédé selon l'une quelconque des revendications 1 à 10 est mélangée à un milieu cosmétiquement acceptable et/ou à un ou plusieurs additifs utilisés en cosmétique.

16. Procédé de traitement cosmétique des matières kératiniques, **caractérisé en ce que** l'on prépare au moins deux compositions cosmétiques selon le procédé défini selon l'une quelconque des revendications 1 à 10, on les mélange et on applique le mélange sur la matière kératinique.

17. Utilisation d'une composition obtenue par le procédé selon l'une quelconque des revendications 1 à 10 pour le traitement cosmétique des matières kératiniques.

18. Utilisation d'une composition obtenue par le procédé selon l'une quelconque des revendications 1 à 10 pour le coiffage des cheveux.

19. Dispositif de conditionnement d'une composition cosmétique, comprenant un logement fermé délimité par au moins une paroi au moins en partie perméable à un fluide sous pression d'au moins 3 bars, la composition contenant au moins un polymère anionique et/ou un polymère non ionique, sous forme solide ou pâteuse.

20. Dispositif selon la revendication 19, **caractérisé en ce que** le logement est délimité par deux feuilles scellées.

21. Dispositif selon la revendication 19, **caractérisé en ce que** le logement est délimité par une barquette fermée par un couvercle.
